# EUROPEAN PATENT APPLICATION

(11) **EP 1 669 076 A1**
(43) Date of publication of application: **14.06.2006**
(21) Application number: 03750244.0
(22) Date of filing: 17.09.2003
(51) Int. Cl.: A61K 31/7008, A61P 1/04

(54) **USE OF N-ACETYL-D-AMINOGLYCOSAMINE IN PREPARATION OF DRUGS FOR MODULATING MICROORGANISMS ON MUCOUS MEMBRANE**

(71) Applicant: Third Military Medical University Chinese People's Liberation Army P.R. of China, Chongqing 400038 (CN); Bio-Wave Institute of Suzhou Hi-Tech New District Corporation, Ltd, Jiangsu 215011 (CN); Beijing Sino-Hongkong Dafu Science & Technology of Biowave Co., Ltd., Beijing 101200 (CN)
(72) Inventor: XU, Qiwang, Shapingba District, Chongqing 400048 (CN); LIU, Junkang, Shapingba District, Chongqing 400038 (CN); YUAN, Zetao, Shapingba District, Chongqing 400038 (CN)
(74) Representative: Bentz, Jean-Paul
(86) International application number: PCT/CN2003/000782
(87) International publication number: WO 2005/025580

(57) **Abstract**

The present invention has disclosed a use of N-acetyl-D-glucosamine in the preparation of a medicament for regulating microecological balance of mucocutaneous membrane. N-acetyl-D-glucosamine has functions of promoting bacteria and cellular redistribution and against allochthonous flora colonization. Preparations comprising N-acetyl-D-glucosamine as main active substance are able to be used for regulating microecological balance of mucocutaneous membrane, and have advantages of significant therapeutic effects, simple manufacture, nonirritant, non-pollution, etc.

## Description

### Technical field

The present invention relates to the use of N-acetyl-D-glucosamine and pharmaceutical acceptable salts thereof in the manufacture of medicaments for regulating microecological balance of mucocutaneous membrane.

### Background Art

At present, the regulation of microecological balance of mucocutaneous membrane is still neglected in China and in foreign countries. Broad-spectrum antibiotics and traditional Chinese medical lotions are usually used in the treatment of diseases associated with mucocutaneous microorganisms, which not only kill or inhibit pathogenic bacteria, but also kill massively normal microbial population and destroy the local microecological balance of mucocutaneous membrane. Diseases associated with the regulation of microecological balance of mucocutaneous membrane are common diseases directly affecting the life quality and include, for example, oral complaints such as halitosis and oral infections; subaxile complaints such as slimy sensation or bromhidrosis; urinary or genital system diseases such as low cleaness of gynecological genitourinary tract, etc. Thus, new agents for regulating microecological balance of mucocutaneous membrane are still in need.

In the research of "bio-waves" theory, the present inventor has set up a bacterial wave growth model. Through research, it is known that this wave is of its intrinsic regulation mechanism: some chemical substances are able to participate the regulation in the bio-wave process, so as to transform an abnormal periodic slow wave into a normal physiological chaotic quick wave, and these kinds of substances are known as promoting wave factors. Through separating, purifying and identifying, it is determined that one of the factors is N-acetyl-D-glucosamine, the promoting wave function of which is shown in regulating the coupled oscillation of cellular membrane proteins and glycolyx. Many biochemical and physiological processes of human body need the participation of the promoting wave factors, and it would lead to an abnormal state, if this kind of promoting wave factors is missing in the living body.

N-acetyl-D-glucosamine is a chemical reagent. From the 1990's, it is continually used to treat periodontitis (WO9102530A1), microbiological infection (W09718790A3), intestinal inflammation (WO9953929A1), cornea disease (JP10287570A2), hypertrophy of the prostate (US05116615) and so on. It is also applied in cosmetology (JP59013708A2), shampoo preparation (JP2011505A2), tissue growth regulation agent (WO/A 8 702244), and etc., but it has not been used in the manufacture of a medicament for regulating microecological balance of mucocutaneous membrane up to now.

### Contents of the invention

The inventor of the present invention surprisingly finds that N-acetyl-D-glucosamine and pharmaceutically acceptable salts thereof are able to regulate microecological balance of mucocutaneous membrane. Because its toxicity and side effects are very light, this compund overcomes the above mentioned deficiencies of drugs in the art.
Thus, the present invention is related to the use of N-acetyl-D-glucosamine and pharmaceutical acceptable salt thereof in the manufacture of a medicament for regulating microecological balance of mucocutaneous membrane.
In addition, the present invention is related to a method for regulating microecological balance of mucocutaneous membrane, including administering to a patient who is in need thereof a preventively or therapeutically effective amount of N-acetyl-D-glucosamine or pharmaceutical acceptable salts thereof.

The molecular formula of N-acetyl-D-glucosamine is C₈H₁₅NO₆, its structure is as follows: 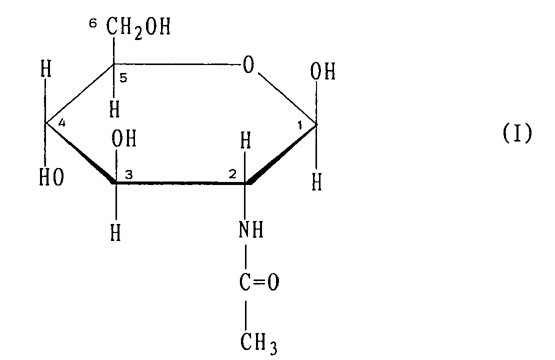 N-acetyl-D-glucosamine can be purchased in market or prepared according to known methods. For instance, patent application WO97/31121 has disclosed a method for preparing N-acetyl-D-glucosamine from chitin by enzyme method, Japanese patent application JP63273493 has disclosed a method in which chitin is partially hydrolyzed into N-acetyl-chitose, and then it is treated with enzyme to obtain N-acetyl-D-glucosamine.

The pharmaceutical acceptable salts of N-acetyl-D-glucosamine that can be mentioned are the salts formed with pharmaceutical acceptable acids, for instance, the salts formed with inorganic acids, such as hydrochloride, hydrobromide, borate, phosphate, sulfate, bisulfate and hydrophosphate, and the salts formed with organic acids, such as citrate, benzoate, ascorbate, methyl sulfate, naphthalene-2-sulfonate, picrate, fumarate, maleate, malonate, oxalate, succinate, acetate, tartrate, mesylate, tosylate, isethionate, α-ketoglutarate, α-glyceryl phosphate and glucose-1-phosphate.
Generally, the compound of the present invention is formulated for topical administration, oral administration or transdermal administration, preferably topical administration and oral administration. In the agent for regulating microecological balance of mucocutaneous membrane according to the method of the present invention, the amount of active component depends on characteristics and severity of diseases to be treated and body weight of patient. The concentration of active component in pharmaceutical preparation is preferably 0.1-10%, and more preferably 0.2-6% by weight. In general, the unit dose is once or many times per day. The total dosage is 10-10,000 mg/day, advantageously 50-5,000mg/day, such as 100-2,000mg/day.
The medicament of the present invention can be formulated to form various dosage forms according to different microecological imbalances to be treated. For example, it can be buccal tablet, sublingual tablet, chewing gum, throat wash, spraying agent, etc., (for instance, a formula of spraying agent comprises: 0.5% N-acetyl-D-glucosamine, 0.01% mint) when it is used for treating oral complaints such as conditions of halitosis and tended to oral infections; it can be topical spraying agent, lotion, aqua, emulsion, cream, ointment, etc. (for instance, a formula of spraying agent comprises: 1% N-acetyl-D-glucosamine, 0.3% sodium benzoate, 10% ethanol) when it is used for deodorizing a large area of body or for treating subaxillary complaints such as slimy sensation or bromhidrosis; and it can also be suppository or aforementioned topical preparations for treating low cleaness of gynecological genitourinary tract and symptoms caused by unknown source of infection.
Solid composition in the form of tablet are made by mixing the main active component with pharmaceutical excipient, such as gelatin, starch, lactose, magnesium stearate, talc powder, Arabic gum and etc. The tablet is able to be coated with sugar or other suitable substances, or making them possess a persistent and delayed function and continually release pre-determined amount of active component.
Topical preparations of the present invention, such as solutions, emulsions, suspensions, viscolloids, creams, ointments, etc. for topically smearing or topically cleaning, can be obtained by mixing active component with one or more pharmaceutically acceptable carriers and additives, such as water, polyethylene glycol, glycerol, Vaseline, xanthan gum, solvents such as alcohols and etc., lubricating agents, adhesives, preservatives, stabilizers, etc., according to known technologies in the art.
Propellants may also be added to form aerosols for topically or whole-body sprinkling.
Suppository is used for genitourinary tract administration, wherein the suppository is prepared by using an adhesive, such as cocoa oil or polyethylene glycol, which melts at genitourinary tract temperature.

Though having no intention to be limited by any theories, the present inventor thinks that the effect of the compound of formula (I) on treating microecological imbalance of mucocutaneous membrane is carried out by regulating the cellular redistribution of organism. The cellular redistribution refers to the continually replacement of the position of the organism cell or the position of the microorganism cell, and rhythmic replacement of gel-sol states of biological macromolecule in cell. N-acetyl-D-glucosamine is able to develop its special efficacy by regulating cellular redistribution of cells in different levels. The macroscopic replacements of cellular position have the wave growth characteristic. Through regulating the wave growth of the organism cell and microorganism cell to be normal, N-acetyl-D-glucosamine makes microorganism cannot be planted locally. In microorganism ecological efficacy, a method which mainly supports normal bacterial colony to grow but not supplement ecological bacterial colony is able to avoid the problem of adaptability to field planting condition existed in the supplementing bacterial colony. In the aspect of repairing skin mucosa tissue, N-acetyl-D-glucosamine has a controlling effect for inflammation, injure, infection, exudation, and this is just the characteristic of the product of the invention which can be widely applied to control the symptom and carry out the treatment to solve the radical problem.

### Optimal embodiment for carrying out the invention

The following experimental examples are used to illustrate that the compound of the present invention (the compound of formula (I)) has promoting wave function, low toxicity, and effectiveness for treating microecological imbalance of mucocutaneous membrane.

### I. Promoting wave test of the compound of formula (I)

### 1. Experimental materials and method:

### 1.1 Samples: pure compound of formula (I)

### 1.2 Experimental materials:

Strain: Proteus Mirabilis (which should comply with the following biological reaction characteristics: dynamics (+), urease (+), lactose (-), glucose (+), H₂S (-), phenylalanine deaminase (+). Culture medium: modified LB culture medium (the component of the composition are: 1% trytones, 0.5% yeast extract, 1% sodium chloride, 0.1% glucose, 0.002% TTC, and pH = 7.2~7.4).

### 1.3 Experimental method:

The Proteus Mirabilis were inoculated at the center of a LB plate, incubated at 37°C and cultured for 9 hours, then concentric rings emerged, extended outward continually with an interval of 3 hours, and this was taken as a control; the compound of formula (I) with final concentration of 0.5 % was added onto a LB plate, and the Proteus Mirabilis were inoculated by the same method and cultured at 37°C, and the results showed that not only the concentric rings emerged with an interval of 3 hours, but also many fine waves on each ring emerged in comparison with the control.

### 2. Experimental results and evaluation:

The experiment adopted a bio-wave model for studying the promoting wave function of the compound of formula (I). It can be seen from the results that the compound of formula (I) was not only able to make bacterial cell reveal a normal bio-wave characteristic, but also to cause the wave to reveal a finer wave mode and shorter wave cycle, and these indicated that the compound of formula (I) has promoting function to bio-waves, and the promoting wave function is able to regulate the cellular redistribution in vivo and is the basis that the compound of formula (I) is used as an regulator for preventing and treating microecological imbalance of mucocutaneous membrane.

### II. Toxicological test of the compound of formula (I), including:

1. Acute toxicity test : including tests of administrating medicine orally, intravenous injection and maximum limit amount for administration;
2. Ames test;
3. Micronucleus test of bone marrow cell of small mouse;
4. Abnormal sexual test for the sperm of mouse;
5. Abnormal aberrance test for the chromosin of mouse' s testis;
6. Chronic lethal test;
7. Subchronic toxicity (feed for 90 days) test;
8. Traditional deformity-inducing test;
The results from these tests showed that in the acute toxicity test of the compound of formula (I), a dosage of more than 2g/kg was taken, which was 300 times greater than the injection dosage for human being, but the acute toxicosis reaction had not appeared. In the long-period toxicity test, the maximum dosage had reached up to 1g/kg, and after the treatment and observation for four weeks, there was no intoxication reaction yet; and in the reproduction test, the mouse was feed from routine dosage of 7mg/kg for 3 generations, and it has been proved that the compound of formula (I) had no influence on the pregnancy, birth, nursing and the growth of baby mouse. So it was proved that the compound of formula (I) was a substance without toxicity.

### III. Tests of bacterial colonization resistance

Pseudomonas aeruginosa is used in the tests of bacterial colonization resistance of the compound of formula (I). The intestinal mucosa of test animals that were pre-administrated with Pseudomonas aeruginosus and were treated by the compound of formula (I) with different concentrations was detected by in situ colonization, qualitative and quantitative methods. The results showed that the colonization resistance of the compound of formula (I) against Pseudomonas aeruginosa increased with the increase of its concentration.

### IV. Clinical trial

56 patients with symptoms of microecological imbalance, including 20 patients with oral complaints, such as halitosis, oral infections caused by clinically unknown pathogenic bacteria, 20 patients with subaxilary complaints, such as slimy sensation or bromidrosis, and 16 patients with low cleaness of gynecological genitourinary tract caused by unknown source of infectious pathogen, were separately treated by mouthwash or topical sprinkle of 1% N-acetyl-D-glucosamine aqueous solution twice daily for 7 days. The improvement of microecology was determined by detecting the microbial population, the total effective rate was 87.8%, and the results are shown below:

**Observation of effects of N-acetyl-D-glucosamine for regulating mucocutaneous microecology**

| | Case number | Cured Effective Ineffective | | | Effective rate (%) |
|---|---|---|---|---|---|
| Oral compliant | 20 | 8 | 10 | 2 | 90 |
| Subaxillary slimy sensation | 10 | 7 | 3 | 0 | 100 |
| Bromidrosis | 10 | 5 | 3 | 2 | 80 |
| Microecological imbalance of gynecological genitourinary tract | 16 | 7 | 6 | 3 | 81.3 |

**Detection results of flora of mucocutaneous microecology regulated by N-acetyl-D-glucosamine (detection rates of various bacteria)**

| Sites | Flora condition before regulation | |
|---|---|---|
| Buccal cavity (represented by salivary bacteria) | Streptococcus mutans (23), streptococcus salivarius (40), Neisser's coccus (50), corynebacterium diphtheroides (18), bacteroides (60), helicoids (12) | Streptococcus mutans (7), streptococcus salivarius (90), Neisser's coccus (95), corynebacterium diphtheroides (8), bacteroides (30), helicoids (0) |
| Skin of axillary fossa | Staphylococci (20), aerobic coryneform bacteria pityrosporum furfur (24), bacillus polymyxa odour (40) | Staphylococci (36), aerobic (18), coryneform bacteria (12), pityrosporum furfur (15), bacillus polymyxa odour (9) |
| Woman's genitourinary tract | Staphylococcus epidermidis (38), bacillus acidi lactici (18), (35), bacillus coli (29) | Staphylococcus epidermidis bacillus acidi lactici (60), bacillus coli (10) |

The detection results in the table showed that after the microecological regulator was used, autochthonous flora increased and allochthonous flora decreased at sites, such as buccal cavity, skin of axillary fossa and woman's genitourinary tract, which indicated that topical microecology was improved.

The present invention develops a new medical use of N-acetyl-D-glucosamine, expands the scope of using N-acetyl-D-glucosamine, and increases the exploitation value of N-acetyl-D-glucosamine. Preparations in various forms comprising N-acetyl-D-glucosamine as active substance are able to be used for regulating microecological balance of mucocutaneous membrane, and have advantages of simple manufacture, nontoxicity and significant therapeutic effects.

## Claims

1. A use of N-acetyl-D-glucosamine and pharmaceutically acceptable salt thereof in the manufacture of a medicament for regulating microecological balance of mucocutaneous membrane.

2. The use according to claim 1, wherein said medicament is of a topical dosage form or a dosage form for systemic administration.

3. The use according to claim 2, wherein said dosage form is aqua, emulsion, spray, cream or slurry.

4. The use according to any one of claims 1 to 3, wherein the concentration of N-acetyl-D-glucosamine in said medicament is 0.1 ~ 10 % by weight.

5. The use according to any one of claims 1 to 3, wherein said medicament has a daily dose of 100~2,000mg of N-acetyl-D-glucosamine.
